(19) Europäisches Patentamt
European Patent Office
Office européen des brevets

(11) EP 1 970 062 A1

(12) **EUROPEAN PATENT APPLICATION**
published in accordance with Art. 158(3) EPC

(43) Date of publication:
**17.09.2008 Bulletin 2008/38**

(21) Application number: **06843451.3**

(22) Date of filing: **27.12.2006**

(51) Int Cl.:
***A61K 31/44*** *(2006.01)* ***A61K 33/30*** *(2006.01)*
***A61P 31/18*** *(2006.01)* ***C07D 213/36*** *(2006.01)*
***C07F 3/06*** *(2006.01)*

(86) International application number:
**PCT/JP2006/326069**

(87) International publication number:
**WO 2007/074871 (05.07.2007 Gazette 2007/27)**

(84) Designated Contracting States:
**AT BE BG CH CY CZ DE DK EE ES FI FR GB GR HU IE IS IT LI LT LU LV MC NL PL PT RO SE SI SK TR**

(30) Priority: **28.12.2005 JP 2005379167**

(71) Applicant: **Kyoto University**
**Kyoto-shi, Kyoto 606-8501 (JP)**

(72) Inventors:
- **FUJII, Nobutaka**
  **Kyoto-shi,**
  **Kyoto 6068501 (JP)**
- **HAMACHI, Itaru**
  **Kyoto-shi,**
  **Kyoto 6158510 (JP)**
- **OJIDA, Akio**
  **Kyoto-shi,**
  **Kyoto 6158510 (JP)**
- **TAMAMURA, Hirokazu**
  **Tokyo 1640011 (JP)**
- **NAKASHIMA, Hideki**
  **Kawasaki-shi,**
  **Kanagawa 2150007 (JP)**

(74) Representative: **Müller-Boré & Partner**
**Patentanwälte**
**Grafinger Strasse 2**
**81671 München (DE)**

# (54) NOVEL CXCR4 ANTAGONIST AND USE THEREOF

(57) The present invention provides a novel CXCR4 antagonist and use thereof.

The present invention provides a novel non-peptidic, low-molecular-weight CXCR4 antagonist using various aromatic compounds each containing a dipicolylamine-zinc complex. The present invention also provides use of the novel CXCR4 antagonist as an anti-HIV agent, a metastasis inhibitor for a malignant tumor, or a chronic rheumatoid arthritis treatment and/or prevention agent.

EP 1 970 062 A1

## Description

TECHNICAL FIELD

The present invention relates to a CXCR4 antagonistic compound and use thereof.

BACKGROUND ART

A chemokine receptor CXCR4 is a seven-transmembrane G-protein-coupled receptor, and is known to transduce the signal of a CXCL12/Stroma cell-derived factor 1 (SDF-1).

A CXCL12-CXCR4 axis is known to be involved in various diseases, including HIV infection, the metastasis/progression of carcinoma cells, and chronic rheumatoid arthritis (RA) (Non-patent Documents 2 to 4).

An ultimate cure for HIV infection or acquired immunodeficiency syndrome (AIDS) has not yet been established, and there is a strong demand for useful medicines that have a new action mechanism. CXCR4 is known as a co-receptor involved in cell entry by T-tropic viruses (Non-patent Document 2).

Carcinoma metastasis is an important factor that has a decisive influence on the patient's future life expectancy. It has been reported that the expression of CXCR4 accelerates in cells such as breast carcinoma cells, and that the expression of its ligand, CXCL12/SDF-1, accelerates in the organs (lymph gland, lung, liver, or bone) where the carcinoma cells metastasize (Non-patent Document 3).

Further, CXCR4 is known to be expressed in malignant cells of at least 23 different kinds of carcinoma, including pancreas carcinoma cells, melanoma cells, prostate carcinoma cells, renal carcinoma cells, neuroblastoma cells, non-Hodgkin's lymphoma cells, small cell lung cancer (SCLC) cells, ovarian carcinoma cells, multiple myeloma cells, chronic lymphatic leukemia (CLL) B-cells, pre-B acuteness lymphoblastic leukemia (ALL) cells and malignant brain tumor cells (Non-patent Document 5).

Chronic rheumatoid arthritis is mainly induced through the accumulation of CD4 positive memory T-cells in an inflammatory synovial membrane. Non-patent Document 4 reports the following. The CXCR4 gene is more actively expressed in the CD4 positive memory T-cells in the articular cavity fluid of a chronic rheumatoid arthritis patient, which facilitates the expression of the CXCL12/SDF-1 gene in the synovial joint membrane tissue. The CXCL12 stimulates the memory T-cells to cause cell movement, thereby inhibiting apoptosis of the T-cells. A CXCL12-CXCR4 axis plays an important role in the accumulation of T-cells in a RA synovial joint membrane.

CXCR4 has thus been considered a target for the treatment of the above-described diseases. Therefore, various CXCR4 antagonists have been developed in the past.

T140 is a peptidic CXCR4 antagonist composed of 14 amino acid residues specifically coupled with CXCR4, and blocks cell invasion by T-tropic HIV-1(X4-HIV-1). 4F-benzoyl-TN14003 and 4F-benzoyl-TE14011 are derivatives of T140, which are more stable in vivo. In animal testing, 4F-benzoyl-TN14003 and 4F-benzoyl-TE14011 exhibited superior properties that inhibit metastasis of certain kinds of carcinoma cells and chronic rheumatoid arthritis, and also showed significant activity in vivo (Non-patent Document 6).

FC131 is a low-molecular-weight lead compound discovered with a cyclic pentapeptide library including $Arg^2$, $Nal^3$, $Tyr^5$ and $Arg^{14}$, which are pharmacophores of T140. The activity of FC131 is comparable to T140.

However, T140 and FC131 are both peptidic compounds that are not suitable for oral administration, and require some processing in the medicine formulation. Therefore, there is a demand for the development of a non-peptidic, low-molecular-weight and highly-active CXCR4 antagonist.

KRH-1636 is known as a non-peptidic, low-molecular-weight compound serving as a CXCR4 antagonist, and is reported to have CXCR4-inhibiting and anti-HIV-1 activity (Non-patent Document 7).

However, KRH-1636 has a long half-life period, and has also been reported to cause safety problems such as accumulation in the body.

In the meantime, Hamachi et al. found that an aromatic compound having a dipicolylamine-zinc complex is useful as a probe for recognizing protein and tyrosyl residues in peptides (Non-patent Document 8 and Patent Document 1).

Patent Document 1: Japanese Unexamined Patent Publication No. 2003-246788
Non-patent Document 1: Journal of Biological Chemistry, Vol.273, Page 4754 (1998)
Non-patent Document 2: Science, Vol.272, Pages 872-877 (1996)
Non-patent Document 3: Nature, Vol.410, Pages 50-56 (2001)
Non-patent Document 4: Journal of Immunology, Vol.165, Pages 6590-6598 (2000)
Non-patent Document 5: Seminars in Cancer Biology, Vol. 14, Pages 171-179 (2004)
Non-patent Document 6: Expert Opinion on Therapeutic Targets, Vol.9, Pages 1267-1282 (2005)
Non-patent Document 7: Proceedings of the National Academy of Sciences, Vol.100, Pages 4185-4190 (2003)

Non-patent Document 8: Journal of American Chemical Society, Vol.124, Pages 6256-6258 (2002)

DISCLOSURE OF THE INVENTION

An object of the present invention is to provide a novel CXCR4 antagonist using a non-peptidic, low-molecular-weight compound. Further, the present invention also provides a novel method for preventing/treating HIV infection and AIDS, carcinoma, and chronic rheumatoid arthritis, by using the CXCR4 antagonist.

Technical Solution

The inventors of the present invention conducted intensive research to solve the foregoing problems, and found that various kinds of aromatic compounds with a dipicolylamine-zinc complex useful as a probe for recognizing protein and tyrosyl residues in peptide have a CXCR4 antagonistic effect and provide a certain effect on the prevention/treatment of HIV infection and AIDS, carcinoma including metastasis, and chronic rheumatoid arthritis. With further research based on these findings, the inventors completed the present invention.
More specifically, the present invention relates to each item of the following inventions.
Item 1. An anti-HIV agent containing, as an active ingredient, at least one member selected from the group consisting of:

(i) a compound or salt thereof, the compound being denoted by General Formula (1a): B'-$CH_2$-A-$CH_2$-B'', wherein A represents a substituted or unsubstituted arylene or a substituted or unsubstituted heteroarylene group, and B' and B'', being the same or different, each represent a group denoted by General Formula (2),

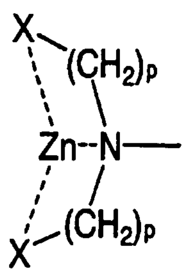
(2)

wherein X represents a substituted or unsubstituted nitrogenous heterocyclic group; and p, being the same or different, each represents 1 to 2; and
(ii) a compound or salt thereof, the compound being denoted by General Formula (1b): B'-$CH_2$-A', wherein A' represents a substituted or unsubstituted aryl or a substituted or unsubstituted heteroaryl group, and B' is the same as that of (1a).

Item 2. A metastasis inhibitor for a malignant tumor containing, as an active ingredient, at least one member selected from the group consisting of:

(i) a compound or salt thereof, the compound being denoted by General Formula (1a): B'-$CH_2$-A-$CH_2$-B'', wherein A represents a substituted or unsubstituted arylene or a substituted or unsubstituted heteroarylene group, and B' and B'', being the same or different, each represent a group denoted by General Formula (2),

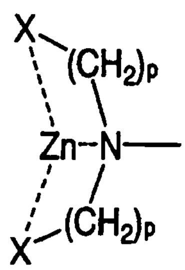
(2)

wherein X represents a substituted or unsubstituted nitrogenous heterocyclic group; and p, being the same or different, each represents 1 to 2; and

(ii) a compound or salt thereof, the compound being denoted by General Formula (1b): B'-$CH_2$-A', wherein A represents a substituted or unsubstituted aryl or a substituted or unsubstituted heteroaryl group, and B' is the same as that of (1a).

Item 3. A chronic rheumatoid arthritis treatment and/or prevention agent containing, as an active ingredient, at least one member selected from the group consisting of:

(i) a compound or salt thereof, the compound being denoted by General Formula (1a): B'-$CH_2$-A-$CH_2$-B'', wherein A represents a substituted or unsubstituted arylene or a substituted or unsubstituted heteroarylene group, and B' and B'', being the same or different, each represent a group denoted by General Formula (2),

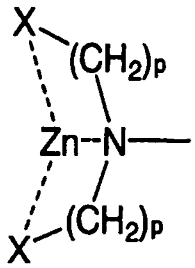

(2)

wherein X represents a substituted or unsubstituted nitrogenous heterocyclic group; and p, being the same or different, each represents 1 to 2; and

(ii) a compound or salt thereof, the compound being denoted by General Formula (1b): B'-$CH_2$-A', wherein A' represents a substituted or unsubstituted aryl or a substituted or unsubstituted heteroaryl group, and B' is the same as that of (1a).

Item 4. A CXCR4 antagonist containing, as an active ingredient, at least one member selected from the group consisting of:

(i) a compound or salt thereof, the compound being denoted by General Formula (1a): B'-$CH_2$-A-$CH_2$-B'', wherein A represents a substituted or unsubstituted arylene or a substituted or unsubstituted heteroarylene group, and B' and B'', being the same or different, each represent a group denoted by General Formula (2),

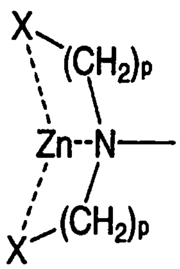

(2)

wherein X represents a substituted or unsubstituted nitrogenous heterocyclic group; and p, being the same or different, each represents 1 to 2; and

(ii) a compound or salt thereof, the compound being denoted by General Formula (1b): B'-$CH_2$-A', wherein A' represents a substituted or unsubstituted aryl or a substituted or unsubstituted heteroaryl group, and B' is the same as that of (1a).

Item 5. A treatment and/or prevention method for a disease whose cause or aggravation relates to CXCR4, comprising administering at least one member selected from the group consisting of:

(i) a compound or salt thereof, the compound being denoted by General Formula (1a): B'-$CH_2$-A-$CH_2$-B'', wherein A represents a substituted or unsubstituted arylene or a substituted or unsubstituted heteroarylene group, and B' and B'', being the same or different, each represent a group denoted by General Formula (2),

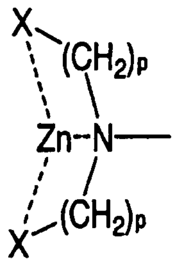
(2)

wherein X represents a substituted or unsubstituted nitrogenous heterocyclic group; and p, being the same or different, each represents 1 to 2; and

(ii) a compound or salt thereof, the compound being denoted by General Formula (1b): B'-$CH_2$-A', wherein A' represents a substituted or unsubstituted aryl or a substituted or unsubstituted heteroaryl group, and B' is the same as that of (1a).

Item 6. The method according to claim 5, wherein the disease relating to CXCR4 is HIV infection.

Item 7. Use of a compound or salt thereof for producing a CXCR4 antagonist, the compound being at least one kind selected from the group consisting of:

(i) a compound or salt thereof, the compound being denoted by General Formula (1a): B'-$CH_2$-A-$CH_2$-B'', wherein A represents a substituted or unsubstituted arylene or a substituted or unsubstituted heteroarylene group, and B' and B'', being the same or different, each represent a group denoted by General Formula (2),

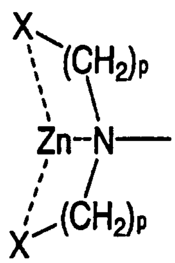
(2)

wherein X represents a substituted or unsubstituted nitrogenous heterocyclic group; and p, being the same or different, each represents 1 to 2; and

(ii) a compound or salt thereof, the compound being denoted by General Formula (1b): B'-$CH_2$-A', wherein A' represents a substituted or unsubstituted aryl or a substituted or unsubstituted heteroaryl group, and B' is the same as that of (1a).

•Compound Denoted by General Formula (1a)

In General Formula (1a), A denotes a substituted or unsubstituted arylene or a substituted or unsubstituted heteroarylene group. When A is a substituted arylene or a substituted heteroarylene group, A generally has 1 to 3 substituents, preferably 1 substituent; however A may have 4 or more substituents.

Examples of the substituent of an arylene or a heteroarylene group include a halogen atom, a nitro group, a cyano group, an amino group, a $C_{1-6}$ alkyl group, and a $C_{1-6}$ alkoxy group.

Examples of the halogen atom include a fluorine atom, a chlorine atom, a bromine atom, and an iodine atom.

Examples of the $C_{1-6}$ alkyl group include methyl, ethyl, n-propyl, isopropyl, n-butyl, isobutyl, tert-butyl, sec-butyl, n-pentyl, neopentyl, n-hexyl, isohexyl, 3-methylpentyl.

Examples of the $C_{1-6}$ alkoxy group include methoxy, ethoxy, n-propoxy, isopropoxy, n-butoxy, isobutoxy, tert-butoxy, sec-butoxy, n-pentoxy, neopentoxy, n-hexyloxy, isohexyloxy, and 3-methylpentoxy.

Preferable examples of the arylene group or heteroarylene group in the substituted or unsubstituted arylene

and substituted or unsubstituted heteroarylene group include phenylene, biphenylene, 2,2'-bipyridinylene, naphthylene, and anthracenylene groups. Phenylene groups are particularly preferable.

In General Formula (1a), B' and B'', being the same or different, each represent a group denoted by General Formula (2). In General Formula (2), X represents a substituted or unsubstituted nitrogenous heterocyclic group. When X is a substituted nitrogenous heterocyclic group having a substituent, X generally has 1 to 3 substituents, preferably 1 substituent; however X may have 4 or more substituents.

In one embodiment of the present invention, the substituted or unsubstituted nitrogenous heterocyclic group is preferably a substituted or unsubstituted nitrogenous heterocyclic group with a ring of a planar structure.

In another embodiment, a substituted or unsubstituted aromatic nitrogenous heterocyclic group is used as the substituted or unsubstituted nitrogenous heterocyclic group of the present invention.

Examples of the nitrogenous heterocyclic group of the substituted or unsubstituted nitrogenous heterocyclic group of the present invention include pyridyl, pyrazinyl, pyrimidyl, pyridazinyl, pyrrolyl, imidazolyl, pyrazolyl, quinolyl, isoquinolyl, indolyl, and isoindolyl.

Preferable examples of the nitrogenous heterocyclic group of the substituted or unsubstituted nitrogenous heterocyclic group of the present invention include a 5 to 6-membered nitrogenous heterocyclic group such as a pyridyl or pyrrolyl group. A 6-membered nitrogenous heterocyclic group such as a pyridyl group is particularly preferable.

A further preferable example of the nitrogenous heterocyclic group of the substituted or unsubstituted nitrogenous heterocyclic group of the present invention is a nitrogenous heterocyclic group having a nitrogen atom at the 2-position, such as a 2-pyridyl or 2-pyrrolyl group.

Examples of the substituent of the nitrogenous heterocyclic group include a halogen atom, a nitro group, a cyano group, an amino group, a $C_{1-6}$ alkyl group, and a $C_{1-6}$ alkoxy group.

In General Formula (2), p, being the same or different, each represents 1 or 2, preferably 1. Note that, "p" in B and "p" in B' may be the same or different.

The relative position between B' and B'' coupled with A in General Formula (1a) is not particularly limited, but they are preferably positioned 180° to each other. For example, when A is a phenylene, biphenylene, or 2,2'-bipyridinylene group, B'- $CH_2$-and B''- $CH_2$- are preferably coupled with A at the para position. When A is a naphthylene group, B'-$CH_2$- and B''-$CH_2$- are preferably coupled at the 1-position and 4-position of the naphthylene group, respectively. When A is an anthracenylene group, B'-$CH_2$- and B''-$CH_2$- are preferably coupled at the 9-position and 10-position of the anthracenylene group, respectively.

•Compound Denoted as Compound (1b)

In General Formula (1b), A' represents a substituted or unsubstituted aryl group or a substituted or unsubstituted heteroaryl group.

Examples of the substituent of the aryl group or the heteroaryl group include a halogen atom, a nitro group, a cyano group, an amino group, a $C_{1-6}$ alkyl group, and a $C_{1-6}$ alkoxy group.

Preferable examples of A' in General Formula (1b) include an anthracenyl group.

"B'" in General Formula (1b) is the same as "B'" in General Formula (1a).

•Salt of the Compound of the Present Invention

The salt of the compound of the present invention is not limited, and can be selected from any pharmaceutically acceptable salt. Typical examples include hydrochloride salts, salts of nitric acid, and perchlorates.

In the present invention, above-mentioned compounds or salts thereof may be used singly or in combination of two or more.

•Production Method of the Compound of the Present Invention

The compound denoted by General Formula (1a) or (1b) of the present invention is publicly known, and may be produced by a publicly known method or any similar method.

The compound of the present invention can be produced by, for example, the method disclosed in Patent Document 1, the method disclosed in Non-patent Document 8, the method described in the following Production Example 1 of the present invention, or any similar method.

•CXCR4 Antagonist of the Present Invention

The compound of the present invention has a strong CXCR4 antagonistic characteristic, and therefore is useful as a CXCR4 antagonist.

•Anti-HIV Agent of the Present Invention

With its strong CXCR4 antagonistic characteristics, the compound of the present invention may be used as an anti-HIV agent.

HIV uses CD4 as a (primary) receptor to infect helper T-cells or macrophages that express CD4, and destroys the cell-mediated immune system. As well as CD4, HIV infection needs a co-receptor that works with the CD4 to facilitate viral invasion of cells. CXCR4 is known as a co-receptor in HIV-1 cell invasion (Non-patent Document 2). The CXCR4 antagonistic characteristics of the compound of the present invention inhibit such HIV-1 invasion of cells, thereby exhibiting an anti-HIV activity.

•Metastasis Inhibitor for a Malignant Tumor According to the Present Invention

With its CXCR4 antagonistic characteristics, the compound of the present invention is useful as a metastasis inhibitor for a malignant tumor.

It is known that the metastasis of some carcinomas relates to the interaction between CXCR4 and the endogenous ligand SDF-1. It is also known that CXCR4 is actively expressed on the cell surfaces of some carcinomas, and that SDF-1 is actively expressed in the organ where the carcinomas cells metastasize. Also, CXCR4 is actively expressed on leukemic cell surfaces, and SDF-1 activates these cells (Non-patent Documents 3 and 5). In view of this, with the above-mentioned CXCR4 antagonistic characteristics, the metastasis inhibitor for a malignant tumor of the present invention is assumed to exhibit an effect that suppresses carcinoma metastasis and an effect that suppresses leukemia progression.

The metastasis inhibitor of the present invention can be used for the treatment of some solid malignant tumors and blood malignant tumors, including pancreas carcinoma, melanoma, prostate carcinoma, renal carcinoma, neuroblastoma, non-Hodgkin's lymphoma, small cell lung cancer (SCLC), ovarian carcinoma, multiple myeloma, chronic lymphatic leukemia (CLL), pre-B acute lymphoblastic leukemia (ALL) and malignant brain tumors.

•Chronic Rheumatoid Arthritis Treatment and/or Prevention Agent of the Present Invention

With its CXCR4 antagonistic characteristic, the compound of the present invention is useful as a chronic rheumatoid arthritis treatment and/or prevention agent.

Chronic rheumatoid arthritis (RA) is mainly induced through the accumulation of CD4 positive memory T-cells in an inflammatory synovial joint membrane. The foregoing non-patent Document 4 reports the following. The CXCR4 gene is more actively expressed in the CD4 positive memory T-cells in the articular cavity fluid of a chronic rheumatoid arthritis patient, facilitating the expression of the CXCL12/SDF-1 gene in the synovial joint membrane. The CXCL12 stimulates the memory T-cells to cause cell movement, thereby inhibiting apoptosis of the T-cells. A CXCL12-CXCR4 axis plays an important role in the accumulation of T-cells in an RA synovial joint membrane. With its CXCR4 antagonistic characteristics, the compound of the present invention suppresses the accumulation of T-cells in the RA synovial joint membrane, thereby exhibiting the effect of treating and/or preventing chronic rheumatoid arthritis.

The CXCR4 antagonist, anti-HIV agent, carcinoma metastasis inhibitor, and chronic rheumatoid arthritis treatment and/or prevention agent of the present invention may be processed into any drug form according to the method of treatment. Examples of drug form include tablets, pills, powder, liquids, suspensions, capsules, suppositories, injectable solutions (liquids or suspensions), sprays, aerosols, respiratory tonics, sustain-release agents, and enteric coating agents. These forms are further categorized into oral agents, parenteral agents, nasal agents, vaginal agents, suppositories, sublingual agents, and ointments, which are formed or prepared using known methods.

The CXCR4 antagonist, anti-HIV agent, carcinoma metastasis inhibitor, and chronic rheumatoid arthritis treatment and/or prevention agent of the present invention may be used with diluent bases, diluents, fillers, extenders, binders, moistening agents, disintegrating agents, disintegration inhibitors, absorbefacients, moisturizers, adsorbents, surfactants, lubricants, buffer agents, isotonizing agents, emulsifiers, suspensions, wetting agents, preservatives or dispersants, that are commonly used according to the drug form.

The formulation of the present invention may contain coloring agents, preservatives, perfumes, flavor agents, sweetening agents, or other medications, as required.

The content of the compound of the present invention in the formulation is not particularly limited; however, the content is typically about 0.01 to 99 wt%, preferably about 10 to 90 wt% based on the whole amount of the formulation.

The method of administering the CXCR4 antagonist, anti-HIV agent, carcinoma metastasis inhibitor, and chronic rheumatoid arthritis treatment and/or prevention agent of the present invention is not particularly limited, and is determined depending on the drug form, the patient's condition including age and sex, the degree of disease, etc. For example, tablets, pills, liquids, suspensions, emulsion, granules, and capsules are administered orally, and the injectable solutions are administered intravenously individually or in combination with a general reinfusion such as glucose or amino acid,

or as required, are individually administered intramuscularly, intradermally, subcutaneously, or intraperitoneally. A suppository is administered intrarectally. A vaginal agent is administered intravaginally. A nasal agent is administered through the nose. A sublingual agent is administered intraorally.

The amount of CXCR4 antagonist, anti-HIV agent, carcinoma metastasis inhibitor, and chronic rheumatoid arthritis treatment and/or prevention agent of the present invention administered is not particularly limited, and can be determined depending on the drug form, the patient's condition including age and sex, the type and degree of disease, etc. The amount is however typically about 0.01 to 50mg/kg/day, preferably about 0.1 to 50mg/kg/day, more preferably about 0.1 to 5mg/kg/day.

The CXCR4 antagonist, anti-HIV agent, carcinoma metastasis inhibitor, or chronic rheumatoid arthritis treatment and/or prevention agent of the present invention requires zinc for its activity expression. Therefore, the compound of the present invention can be easily inactivated by administering a chelating agent such as EDTA, or a thiol compound having a chelating effect or the like to remove zinc. Further, the compound of the present invention may be inactivated by masking the zinc complex using a pyrophosphoric acid or polyphosphoric acids such as ATP.

Further, the present invention provides a method for treating diseases caused or aggravated by a CXCR4-related factor, the method comprising administering the foregoing compound (or a salt thereof) to mammals including humans. The diseases caused or aggravated by a CXCR4-related factor designate diseases developed or aggravated by a factor related to a CXCR4 (chemokine receptor)-controlling signal transduction system. Such diseases or disorders include HIV infection, acquired immunodeficiency syndrome (AIDS), chronic rheumatoid arthritis, burn injuries, and bedsores. The method of the present invention can also be used to suppress the metastasis of malignant tumors. The details of the compound (e.g. compounds, administration amount, etc.) used as an active ingredient in the treatment method of the present invention are the same as those mentioned above.

## EFFECT OF THE INVENTION

The non-peptidic compound of the present invention has a strong CXCR4 antagonistic effect.

The anti-HIV agent of the present invention inhibits the coupling between CXCR4 and CXCL12/SDF-1, thereby exhibiting an effect for treating HIV infection and AIDS.

The present invention's metastasis inhibitor for malignant tumors inhibits the coupling between CXCR4 and CXCL12/SDF-1, thereby suppressing the metastasis of malignant tumors.

The treatment and/or prevention agent for chronic rheumatoid arthritis of the present invention inhibits the coupling between CXCR4 and CXCL12/SDF-1, thereby exhibiting a treatment/prevention effect for chronic rheumatoid arthritis.

Further, in addition to the foregoing diseases, the CXCR4 antagonist of the present invention is also assumed to be effective for other conditions considered to be related to CXCR4 (e.g., burn injuries and bedsores).

The compound of the present invention can be produced by any publicly known method with a significantly short and easy process at low cost. Therefore, the present invention provides an anti-HIV agent, a metastasis inhibitor for malignant tumors, a treatment method and/or drug for chronic rheumatoid arthritis, and a CXCR4 antagonist at low cost.

The compound of the present invention decreases the antagonistic activity of CXCR4 by losing the zinc atom that forms the complex. Therefore, if, for example, a problematic side effect occurs in the object under continuous administration of the compound of the present invention, detoxification can be accomplished immediately using a chelating agent or the like, inactivating the effect of the drug.

## BEST MODE FOR CARRYING OUT THE INVENTION

•Production of Compound

The following describes an example method for synthesizing a compound of the present invention.

•Production Example 1: Production of bis(2,2'-dipicolylamino)-p-xylene/zinc complex

1. Synthesis of bis(2,2'-dipicolylamino)-p-xylene

P-xylenediamine (1 Eq.) and pyridine-2-carbaldehyde (10 Eq.) were added to ClCH in $CH_2Cl$/dimethyl formamide (9:1) at room temperature, and NaBH $(OAc)_3$ was further added at 0˚C. The reaction proceeded for 5 hours at room temperature. The degree of progress of the condensation reaction was measured with an analytical HPLC (COSMOSIL 5C18 AR-II column: acetonitrile-water) and the ion-spray mass spectrum assay. After the resulting solution was diluted by distilled water, the aqueous solution was purified with a large separation column HPLC (COSMOSIL 5C18 AR-II column: acetonitrile-water) to obtain a single-peak, followed by freeze-drying. 4M HCl/dioxane was added to the

residue before carrying out reduced-pressure distillation. The residue was further mixed with ether, and allowed to stand for 5 hours at -20°C. The precipitated crystals (hydrochloride salt) were filtered to obtain bis(2,2'-dipicolylamino)-p-xylene. The purity was confirmed by HPLC.

2. Synthesis of bis(2,2'-dipicolylamino)-p-xylene/zinc complex

4M NaOH aqueous solution and acetic acid ethyl were added to bis (2,2'-dipicolylamino)-p-xylene hydrochloride salt at 0°C. The acetic acid ethyl layer was separated and washed with water, and dried with anhydrous $MgSO_4$ to distill off the solvent under reduced pressure. The obtained hydrochloride salt-free bis (2,2'-dipicolylamino)-p-xylene was dissolved in methanol/tetrahydrofuran (8:1), and a 0.5M $Zn(NO_3)_2$ aqueous solution (2.1eq) was added dropwise to the solution at room temperature, and stirred overnight. The resulting solution was concentrated under reduced pressure, followed by freeze-drying. The residue was washed with water, and was recrystallized by methanol-acetic acid ethyl to obtain a bis(2,2'-dipicolylamino)-p-xylene/zinc complex (Compound (1c)). The zinc complex was confirmed by way of the ion-spray mass spectrum.

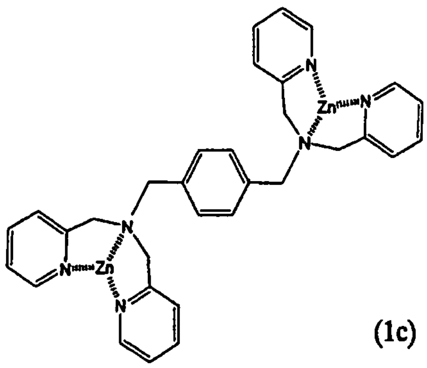

(1c)

•Production example 2: Production of 9,10-bis[(2,2'-dipicolylamino) methyl]anthracene/Zn complex

In accordance with Non-patent Document 8, 9, 10-bis[(2,2'-dipicolylamino) methyl]anthracene/zinc complex was produced.

Experiment Example 1: CXCR4 antagonistic activity

Various aromatic compounds each having a dipicolylamine-zinc complex were prepared, and their CXCR4 antagonistic effects were examined using the following method.

A CXCR4 transfected Chinese hamster ovary(CHO) cells ($3 \times 10^4$ cell/100μL/well) were plated in each well of a flat-bottomed micro-titer tray. The samples were incubated in a $CO_2$ incubator at 37°C, and the cells were loaded in Ham F-12 buffer(80μL/well) for an hour at 37°C, together with 5pM Fura-2AM (Dojindo, Kumatomo, Japan), 2.5mM Probenecid (Sigma) and 20mM HEPES(pH7.4).

Thereafter, the cells were washed twice with Hank's balanced salt solution (100μL×2), and placed in a spectrofluorimeter (96 well Fluorescence Drug Screening System, Hamamatsu Photonics K.K. Japan). 30 seconds after the measurement started, the cells were incubated in Hank's balanced salt solution with plural test compounds of different concentrations. After 3 minutes, recombinant SDF-1a (PreproTech, 30nM/40mL/well) was added.

Changes of the CXCR4 transfect CHO cell system loaded with Fura2-A$[Ca^{2+}]_i$ were recorded in real time with a modification method of Fura-2. The CXCR4 antagonistic effect was measured based on the inhibition of $Ca^{2+}$ movement induced by the stimulation of SDF-1a via CXCR4 ($IC_{50}$).

Tables 1 to 3 show structures of the test objects and their CXCR4 antagonistic activities.

Table 1

| Reference Example Compound | Structure | $IC_{50}$(μM) |
|---|---|---|
| I | H-Arg-Arg-Nal-Cys-Tyr-Arg-Lys-D-Lys-Pro-Tyr-Arg-Cit-Cys-Arg-OH | 0.0031 |
| II | cyclo(-Nal-Gly-D-Tyr-Arg-Arg-) | 0.0055 |

(continued)

| Reference Example Compound | Structure | $IC_{50}$(μM) |
|---|---|---|
| III | 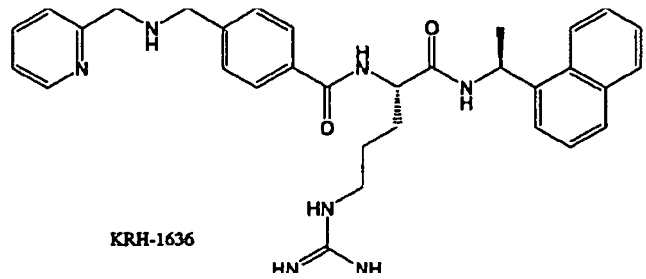 KRH-1636 | 0.24 |

Table 2

| Exmple Compound | Structure | $IC_{50}$(μM)[a] |
|---|---|---|
| 1 | 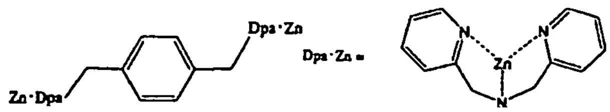 | 0.10 |
| 2 | 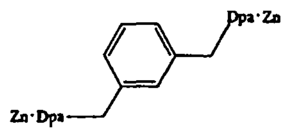 | 0.49 |
| 3 | 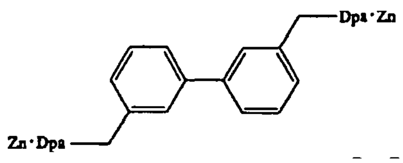 | 0.35 |
| 4 | 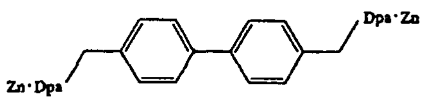 | 0.12 |
| 5 | 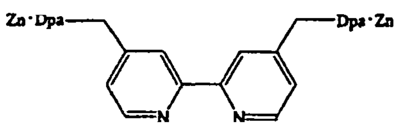 | 0.46 |
| 6 | 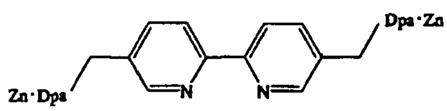 | 0.10 |
| 7 | 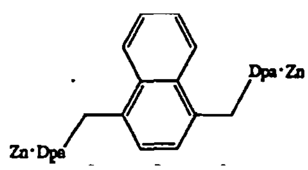 | 0.18 |
| 8 | 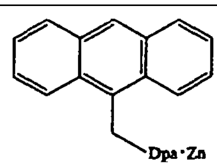 | 0.77 |

(continued)

| Exmple Compound | Structure | IC$_{50}$(μM)[a] |
|---|---|---|
| 9 | 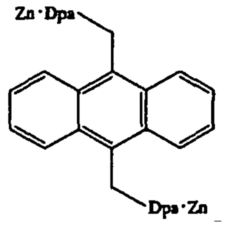 | 0.24 |
| 10 | 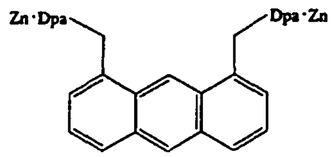 | 0.75 |

Table 3

| Comparative Example Compound | Structure | IC$_{50}$(μM) |
|---|---|---|
| A | Compound resulting from removal of zinc from Compound of Example 1 | >10 |
| B | Compound resulting from removal of zinc from Compound of Example 4 | >10 |
| C | Compound resulting from removal of zinc from Compound of Example 6 | >10 |
| D | Compound resulting from removal of zinc from Compound of Example 9 | >10 |

The positive controls, Reference Example Compound I (T140), Reference Example Compound II (FC131) and Reference Example Compound III (KRH-1636), showed strong CXCR4 antagonistic activities.

Example Compound 1, having two sets of 2,2'-dipicolylamine-zinc complex units (Dpa/Zn units, hereinafter) at the para position in the benzene ring, showed strong CXCR4 antagonistic activity. Example Compound 2, having two sets of Dpa/Zn units at the meta position in the benzene ring, showed weaker activity than that of Example Compound 1.

The biphenyl compounds (Example Compounds 3 and 4), having Dpa/Zn units at the 3,3'position and the 4,4' position, respectively, both showed significantly high CXCR4 antagonistic activity. However, Example Compound 4 showed stronger activity than Example Compound 3.

Further, no critical difference in CXCR4 antagonistic activity was found between Example Compounds 5 and 6, in which the biphenyl unit of Example Compounds 3 and 4 was replaced with [2,2']bipyridyl. The naphthalene compound (Example Compound 7) and the anthracene compound (Example Compound 9), having Dpa/Zn units at the 1,4'position and the 9,10' position, respectively, both showed high activity. Example Compound 8, having only one Dpa/Zn unit, and the anthracene compound (Example Compound 10), having two sets of Dpa/Zn units at the 1,8 position, showed sufficient, though not significantly high, CXCR4 antagonistic activity.

In contrast, Comparative Example Compounds A to D, in which the zinc of Example Compounds 1, 4, 6 and 9 was removed, did not show any significant activity until 10μM. Experiment example 2: CXCR4 binding activity Example Compounds 1, 4, 6, and 7, which showed strong CXCR4 antagonistic activity in Experiment Example 1, were examined for their CXCR4 binding activity using the following method.

A CXCR4 transfected CHO cells suspended in Ham's F-12 buffer (0.5% bovine serum albumin, 20mM HEPES buffer) were placed in a silicon-coated test tube ($5 \times 10^5$cells/120μL/well). Cold SDF-1 a (Final concentration 0.1μM, 15μL/well) and a test object having one of various concentrations (15μL/well) was added to the test tube. Thereafter, $^{125}$I-SDF-1 a (PerkinElmer, final concentration = 0.1nM, 15μL/well) was added. The sample was incubated for an hour at 0 ˚C, mixed with oil (dibutyl phthalate: olive oil=4:1(v/v), 500μL/well), then centrifuged for 2 minutes at 14,000rpm. After removing the water layer and the organic layer, the bottom part was separated from the test tube and was placed in a radioimmunoassay counting tube. Thereafter, count per minute (CPM) was measured with a γ-counter. The inhibition

rate of the test compound with respect to $^{125}$I-SDF-1 a bonding was found by the following equation. $IC_{50}$ denotes a concentration of a test object whose inhibition rate is 50%.

$$\text{Inhibition rate (\%)} = (Et - Ea)/(Et - Ec) \times 100$$

Et: amount of radiation in a sample that does not contain the test object
Ec: amount of radiation in a sample that contains cold SDF-1 a as a test sample
Ea: amount of radiation in a sample that contains a test sample

Table 4 shows the results. These compounds all exhibit CXCR4 bonding activity. In particular, the CXCR4 bonding activity of Compound 1 was comparative to that of Reference Example Compound III (KRH-1636).

Table 4

| Compound | $IC_{50}$(μM)[a] |
|---|---|
| Compound of Example 1 | 0.047 |
| Compound of Example 4 | 0.18 |
| Compound of Example 6 | 0.22 |
| Compound of Example 7 | 0.42 |
| Compound of Example I (T140) | 0.00093 |
| Compound of Example II (FC131) | 0.0030 |
| Compound of Example III (KRH-1636) | 0.034 |

Experiment Example 3: Anti-HIV activity

Using the following method, an anti-HIV-1 activity was examined using Example Compound 1, which is most desirable and simplest among the Example Compounds. The method carries out measurement of anti-HIV-1 activity based on the inhibition effect with respect to cellular degeneration induced in the MT-4 cells by HIV-1.

(1) Cell Culture

MT-4 and MOLT-4 cells, human T-cell systems, are incubated in a RPMI1640 culture medium containing 10% heat-inactivated fetal bovine serum, 100IU/mL of penicillin, and 100mg/mL of streptomycin.

(2) Virus

HIV-$1_{IIIB}$, an X4-HIV-1-strain, was used as virus. The virus was obtained from a culture solution supernatant of MOLT-4/HIV-$1_{IIIB}$ cells persistently infected with HIV-1, and was kept at -80°C before use.

(3) Assay

Plural test compounds of different concentrations were individually added to HIV-1 infected MT-4 cells (virus number/cells number=0.01), and the samples were plated in the wells of a flat-bottomed micro-titer tray ($1.5 \times 10^4$cells/well). The samples were incubated in a $CO_2$ incubator for five days at 37°C. A viable cell count was carried out ($EC_{50}$) with an MTT method.

Example Compound 1 showed superior inhibition activity against cellular degeneration induced in the MT-4 cells by HIV-1 (EC50=7.1μM).

## Claims

**1.** An anti-HIV agent containing, as an active ingredient, at least one member selected from the group consisting of:

(i) a compound or salt thereof, the compound being denoted by General Formula (1a):

B'- $CH_2$-A- $CH_2$-B'',

wherein A represents a substituted or unsubstituted arylene or a substituted or unsubstituted heteroarylene group, and B' and B'', being the same or different, each represent a group denoted by General Formula (2),

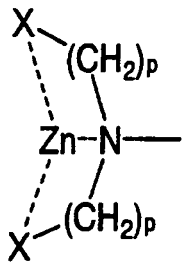

(2)

wherein X represents a substituted or unsubstituted nitrogenous heterocyclic group; and p, being the same or different, each represents 1 to 2; and
(ii) a compound or salt thereof, the compound being denoted by General Formula (1b):

B'- $CH_2$-A',

wherein A' represents a substituted or unsubstituted aryl or a substituted or unsubstituted heteroaryl group, and B' is the same as that of (1a).

**2.** A metastasis inhibitor for a malignant tumor containing, as an active ingredient, at least one member selected from the group consisting of:

(i) a compound or salt thereof, the compound being denoted by General Formula (1a):

B'- $CH_2$-A- $CH_2$-B'',

wherein A represents a substituted or unsubstituted arylene or a substituted or unsubstituted heteroarylene group, and B' and B'', being the same or different, each represent a group denoted by General Formula (2),

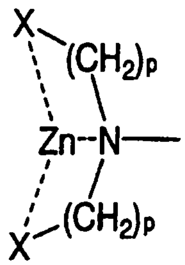

(2)

wherein X represents a substituted or unsubstituted nitrogenous heterocyclic group; and p, being the same or different, each represents 1 to 2; and
(ii) a compound or salt thereof, the compound being denoted by General Formula (1b):

B'- $CH_2$-A',

wherein A' represents a substituted or unsubstituted aryl or a substituted or unsubstituted heteroaryl group, and B' is the same as that of (1a).

**3.** A chronic rheumatoid arthritis treatment and/or prevention agent containing, as an active ingredient, at least one member selected from the group consisting of:

(i) a compound or salt thereof, the compound being denoted by General Formula (1a):

$B'- CH_2-A- CH_2-B''$,

wherein A represents a substituted or unsubstituted arylene or a substituted or unsubstituted heteroarylene group, and B' and B'', being the same or different, each represent a group denoted by General Formula (2),

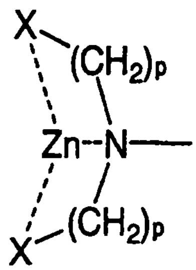

(2)

wherein X represents a substituted or unsubstituted nitrogenous heterocyclic group; and p, being the same or different, each represents 1 to 2; and

(ii) a compound or salt thereof, the compound being denoted by General Formula (1b):

$B'- CH_2-A'$,

wherein A' represents a substituted or unsubstituted aryl or a substituted or unsubstituted heteroaryl group, and B' is the same as that of (1a).

**4.** A CXCR4 antagonist containing, as an active ingredient, at least one member selected from the group consisting of:

(i) a compound or salt thereof, the compound being denoted by General Formula (1a):

$B'- CH_2-A- CH_2-B''$,

wherein A represents a substituted or unsubstituted arylene or a substituted or unsubstituted heteroarylene group, and B' and B'', being the same or different, each represent a group denoted by General Formula (2),

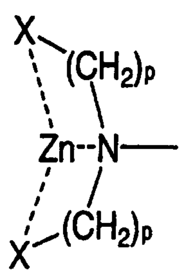

(2)

wherein X represents a substituted or unsubstituted nitrogenous heterocyclic group; and p, being the same or different, each represents 1 to 2; and

(ii) a compound or salt thereof, the compound being denoted by General Formula (1b):

$B'- CH_2-A'$,

wherein A' represents a substituted or unsubstituted aryl or a substituted or unsubstituted heteroaryl group, and B' is the same as that of (1a).

**5.** A treatment and/or prevention method for a disease whose cause or aggravation relates to CXCR4, comprising administering at least one member selected from the group consisting of:

(i) a compound or salt thereof, the compound being denoted by General Formula (1a):

$$B'\text{-}CH_2\text{-}A\text{-}CH_2\text{-}B'',$$

wherein A represents a substituted or unsubstituted arylene or a substituted or unsubstituted heteroarylene group, and B' and B'', being the same or different, each represent a group denoted by General Formula (2),

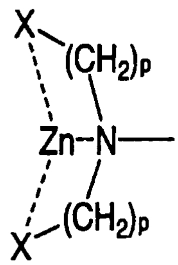

(2)

wherein X represents a substituted or unsubstituted nitrogenous heterocyclic group; and p, being the same or different, each represents 1 to 2; and
(ii) a compound or salt thereof, the compound being denoted by General Formula (1b):

$$B'\text{-}CH_2\text{-}A',$$

wherein A' represents a substituted or unsubstituted aryl or a substituted or unsubstituted heteroaryl group, and B' is the same as that of (1a).

**6.** The method according to claim 5, wherein the disease relating to CXCR4 is HIV infection.

**7.** Use of a compound or salt thereof for producing a CXCR4 antagonist, the compound being at least one member selected from the group consisting of:

(i) a compound or salt thereof, the compound being denoted by General Formula (1a):

$$B'\text{-}CH_2\text{-}A\text{-}CH_2\text{-}B'',$$

wherein A represents a substituted or unsubstituted arylene or a substituted or unsubstituted heteroarylene group, and B' and B'', being the same or different, each represent a group denoted by General Formula (2),

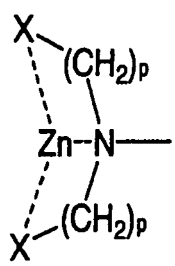

(2)

wherein X represents a substituted or unsubstituted nitrogenous heterocyclic group; and p, being the same or different, each represents 1 to 2; and
(ii) a compound or salt thereof, the compound being denoted by General Formula (1b):

$B'- CH_2-A'$,

wherein A' represents a substituted or unsubstituted aryl or a substituted or unsubstituted heteroaryl group, and B' is the same as that of (1a).

**INTERNATIONAL SEARCH REPORT**

International application No.
PCT/JP2006/326069

A. CLASSIFICATION OF SUBJECT MATTER
*A61K31/44*(2006.01)i, *A61K33/30*(2006.01)i, *A61P31/18*(2006.01)i, *C07D213/36*(2006.01)n, *C07F3/06*(2006.01)n

According to International Patent Classification (IPC) or to both national classification and IPC

B. FIELDS SEARCHED

Minimum documentation searched (classification system followed by classification symbols)
A61K31/44-A61K31/444, A61K33/30, C07D213/24-C07D213/89, C07F3/06

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2007 |
| Kokai Jitsuyo Shinan Koho | 1971-2007 | Toroku Jitsuyo Shinan Koho | 1994-2007 |

Electronic data base consulted during the international search (name of data base and, where practicable, search terms used)
CAplus/REGISTRY/BIOSIS/MEDLINE/WPIDS(STN)

C. DOCUMENTS CONSIDERED TO BE RELEVANT

| Category* | Citation of document, with indication, where appropriate, of the relevant passages | Relevant to claim No. |
|---|---|---|
| Y | JP 2001-253871 A (Japan Science and Technology Corp.), 18 September, 2001 (18.09.01), (Family: none) | 1-7 |
| Y | Gerlach, LO. et al. Metal ion enhanced binding of AMD3100 to $Asp^{262}$ in the CXCR4 receptor. Biochemistry. 2003, Vol.42, No.3, pp.710-717. | 1-7 |

☐ Further documents are listed in the continuation of Box C.  ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 29 January, 2007 (29.01.07) | 06 February, 2007 (06.02.07) |

| Name and mailing address of the ISA/ | Authorized officer |
|---|---|
| Japanese Patent Office | |
| Facsimile No. | Telephone No. |

Form PCT/ISA/210 (second sheet) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2003246788 A


### Non-patent literature cited in the description


- *Journal of Biological Chemistry,* 1998, vol. 273, 4754
- *Science,* 1996, vol. 272, 872-877
- *Nature,* 2001, vol. 410, 50-56
- *Journal of Immunology,* 2000, vol. 165, 6590-6598
- *Seminars in Cancer Biology,* 2004, vol. 14, 171-179
- *Expert Opinion on Therapeutic Targets,* 2005, vol. 9, 1267-1282
- *Proceedings of the National Academy of Sciences,* 2003, vol. 100, 4185-4190
- *Journal of American Chemical Society,* 2002, vol. 124, 6256-6258